# EUROPÄISCHE PATENTANMELDUNG

(11) **EP 2 602 327 A1**
(43) Veröffentlichungstag der Anmeldung: **12.06.2013**
(21) Anmeldenummer: 11009622.9
(22) Anmeldetag: 06.12.2011
(51) Int. Cl.: C12P 5/02

(54) **Verfahren zur Erzeugung von Biogas aus Biomasse sowie Biogasanlage**

(71) Anmelder: Niederbacher, Michael, 39031 Bruneck (IT)
(72) Erfinder: Niederbacher, Michael, 39031 Bruneck (IT)
(74) Vertreter: Liebl, Thomas

(57) **Zusammenfassung**

Die Erfindung betrifft ein Verfahren zur Erzeugung von Biogas aus Biomasse, bei dem die Biomasse (20) in einem ersten Schritt einer Aufschlusseinrichtung (2) zugeführt wird, in der ein Zellaufschluss erfolgt und die zugeführte Biomasse aufgeschlossen wird, wobei anschließend die aufgeschlossene Biomasse einer ersten Hydrolyseeinrichtung (6) zugeführt wird, in der die aufgeschlossene Biomasse hydrolysiert wird. Anschließend wird die in der ersten Hydrolyseeinrichtung (6) hydrolysierte Biomasse einer ersten Separatoreinrichtung (8) zugeführt, in der eine Auftrennung in eine feststoffhaltige Feststoffphase und in eine Flüssigphase durchgeführt wird. Die Feststoffphase wird einer zweiten Hydrolyseeinrichtung (11) zugeführt, in der die Feststoffphase hydrolysiert wird, wobei anschließend die in der zweiten Hydrolyseeinrichtung (6) hydrolysierte Biomasse einer zweiten Separatoreinrichtung (15) zugeführt wird, in der eine zweite Feststoffphase und eine zweite Flüssigphase abgetrennt wird. Sowohl die mittels der ersten Separatoreinrichtung (8) abgetrennte erste Flüssigphase als auch die mittels der zweiten Separatoreinrichtung (15) abgetrennte zweite Flüssigphase wird einem Endlager und/oder Fermenter (13) zugeführt, aus dem bevorzugt ein Rezirkulat (16) abgezogen und der ersten Hydrolyseeinrichtung (6) und/oder der zweiten Hydrolyseeinrichtung (11) mittelbar und/oder unmittelbar zugeführt wird. Zudem wird eine Biogasanlage zur Durchführung eines derartigen Verfahrens beansprucht.

## Beschreibung

Die Erfindung betrifft ein Verfahren zur Erzeugung von Biogas aus Biomasse nach dem Oberbegriff des Anspruchs 1 sowie eine Biogasanlage zur Erzeugung von Biogas nach dem Oberbegriff des Anspruchs 15.

Es ist allgemein bekannt, durch den Einsatz von Biomasse in Fermentern einer Biogasanlage Biogas zu erzeugen, aus dem dann wiederum elektrische Energie gewonnen wird. Bei der eingesetzten Biomasse handelt es sich regelmäßig um nachwachsende Rohstoffe, die landwirtschaftlich angebaut werden. Der Begriff Biomasse umfasst aber auch Reststoffe aus der landwirtschaftlichen Produktion, wie zum Beispiel Gülle, Hühnermist oder dergleichen. Auch organisch abbaubare bzw. fermentierbare Abfälle, wie zum Beispiel Schlachtabfälle oder Klärschlämme, um nur einige Beispiele zu nennen, werden als Biomasse in Biogasanlagen eingesetzt und vergärt.

Insbesondere bei der Nutzung von nachwachsenden Rohstoffen als Biomasse zur Biogaserzeugung verhindert deren chemische Struktur eine vollständige Umsetzung in Biogas. Der Grund liegt darin, dass wesentliche Bestandteile des pflanzlichen Materials aus für Mikroorganismen schwer oder gar nicht zugänglicher Cellulose, Hemicellulose und Lignin bestehen, was bei der Anwendung herkömmlicher Vergärungstechnik in Biogasanlagen, welche außer einer mechanischen Grobzerkleinerung des eingesetzten Biomassematerials keine weitergehende Substratbehandlung vorsieht, regelmäßig zu unbefriedigenden und damit auch unwirtschaftlichen Zersetzungsergebnissen bzw. zu einem unerwünscht niedrigen Gesamtwirkungsgrad der Biogasanlage führt, Um unter diesen Bedingungen eine einigermaßen zufriedenstellende Biogasausbeute zu erzielen, sind Verweilzeiten der Substrate in anaeroben Fermentern von 50 bis 150 Tagen üblich, was ersichtlich relativ langwierig ist. Ein weiterer wesentlicher Nachteil dieser hohen Verweilzeiten der Substrate in den Fermentern ist, dass die Fermenter entsprechend großvolumig auszulegen sind, damit über die Menge der vergorenen Biomasse eine einigermaßen zufriedenstellende Biogasausbeute erzielt werden kann. Derartige großvolumige Behälter sind jedoch relativ teuer und benötigen zudem viel Bauraum.

Ein weiterer Nachteil herkömmlicher Biogasanlagen ist, dass bei in Reihe geschalteten Fermentern jeweils stets nur der erste Fermenter zufriedenstellend arbeitet und im Wesentlichen voll ausgelastet ist, da in diesem der größte Anteil der mikrobiologisch verfügbaren organischen Stoffe schnell und frühzeitig umgesetzt wird. Alle nachgeschalteten Fermenter sind allerdings in ihrer Abbauleistung und Geschwindigkeit stark begrenzt, was vor allem in der sehr langsamen Hydrolyse der verbliebenen organischen Fraktionen liegt. Dieser die Geschwindigkeit limitierende Abbauschritt führt daher zu einer deutlichen Verlangsamung des Methanbildungsprozesses.

Um die Verweilzeit der Substrate in den Fermentern zu verringern bzw. die Abbaugeschwindigkeit bei der Biogasbildung in den Fermentern zu erhöhen, ist es bereits allgemein bekannt, die den Fermentern zugeführte Biomasse (insbesondere nachwachsende Rohstoffe) mechanisch und/oder physikalisch und/oder chemisch und/oder biologisch vorzubehandeln bzw. aufzubereiten. Im Rahmen dieser Aufbereitung erfolgt, zum Beispiel durch Extruder, ein wenigstens teilweiser Zellaufschluss, wodurch im Rahmen der nachfolgenden Fermentation im Fermenter eine Erhöhung der Abbaugeschwindigkeit erzielt und damit der Wirkungsgrad der Biogasanlage insgesamt erhöht werden kann. Um den Wirkungsgrad einer derartigen Biogasanlage noch weiter zu erhöhen, ist es aus der gattungsbildenden WO 2009/016082 A2 weiter bekannt, den Gärrest aus einem ersten Fermenter einer Separatoreinrichtung zuzuführen, in der eine Auftrennung in eine feststoffhaltige Feststoffphase und in eine Flüssigphase erfolgt. Die so abgetrennte Feststoffphase wird anschließend einer Thermodruckhydrolyse bei Temperaturen von mindestens 170°C und Drücken von mindestens 1,0 MPa unterworfen, wobei die so behandelte Feststoffphase entweder in den ersten anaeroben Fermenter rückgeführt wird oder einem zweiten anaeroben Fermenter zugeführt wird und dort einem weiteren Gärprozess unterworfen wird. Im Falle eines zweiten Fermenters wird diesem auch die insbesondere aus Mikroorganismen und Wasser bestehende Flüssigphase der Phasentrennung zugeführt. Mit einer derartigen Verfahrensführung soll die Gesamtverweilzeit der Biomasse auf unter 50 Tage verkürzt werden können und gleichzeitig die Quantität als auch die Qualität des hergestellten Biogases verbessert werden. Zudem sollen aufgrund der deutlich gesteigerten Abbaugeschwindigkeit die bestehenden Fermenterbehältervolumina wesentlich effektiver ausgenutzt werden können und neu zu planende Anlagen dementsprechend kleiner dimensioniert werden können.

Dementsprechend ist es Aufgabe der vorliegenden Erfindung, ein Verfahren zur Erzeugung von Biogas aus Biomasse, insbesondere aus nachwachsenden Rohstoffen mit oder ohne Co-Substraten (wie zum Beispiel landwirtschaftliche Reststoffe und/oder Klärschlämme und/oder Prozesswässer), zur Verfügung zu stellen, mittels dem bei gleichbleibender Biogasausbeute die Gesamtverweilzeit der Biomasse in der Biogasanlage noch weiter reduziert werden kann bzw. mittels dem eine noch höhere Biogasausbeute erzielt werden kann. Ferner ist es eine weitere Aufgabe der vorliegenden Erfindung, eine Biogasanlage zur Erzeugung von Biogas aus Biomasse zur Verfügung zu stellen, mit der ebenfalls eine Lösung für die zuvor in Verbindung mit dem Verfahren genannte Aufgabenstellung erzielt werden kann.

Die Lösung dieser Aufgabe gelingt mit den Merkmalen der unabhängigen Patentansprüche. Vorteilhafte Ausgestaltungen hierzu sind jeweils Gegenstand der darauf rückbezogenen Unteransprüche.

Gemäß Anspruch 1 wird ein Verfahren zur Erzeugung von Biogas aus Biomasse vorgeschlagen, bei dem die Biomasse in einem ersten Schritt einer Aufschlusseinrichtung zugeführt wird, in der ein Zellaufschluss erfolgt und die zugeführte Biomasse wenigstens teilweise aufgeschlossen wird. Anschließend wird die aufgeschlossene Biomasse wenigstens zum Teil einer ersten Hydrolyseeinrichtung zugeführt, in der die aufgeschlossene Biomasse wenigstens teilweise hydrolysiert wird. Anschließend wird die in der ersten Hydrolyseeinrichtung wenigstens teilweise hydrolysierte Biomasse wenigstens zum Teil einer ersten Separatoreinrichtung zugeführt, in der eine Auftrennung in eine feststoffhaltige Feststoffphase und in eine Flüssigphase durchgeführt wird, wobei die Feststoffphase wenigstens zum Teil einer zweiten Hydrolyseeinrichtung zugeführt wird, in der die Feststoffphase wenigstens teilweise hydrolysiert wird. Anschließend wird die in der zweiten Hydrolyseeinrichtung wenigstens teilweise hydrolysierte Biomasse wenigstens zum Teil einer zweiten Separatoreinrichtung zugeführt, in der eine zweite Feststoffphase und eine zweite Flüssigphase abgetrennt wird, wobei sowohl die mittels der ersten Separatoreinrichtung abgetrennte erste Flüssigphase als auch die mittels der zweiten Separatoreinrichtung abgetrennte zweite Flüssigphase wenigstens zum Teil einem Endlager und/oder Fermenter zugeführt wird, aus dem zu definierten Zeiten und/oder in definierter Menge ein Rezirkulat abgezogen und der ersten Hydrolyseeinrichtung und/oder der zweiten Hydrolyseeinrichtung mittelbar und/oder unmittelbar zugeführt wird.

Die Begrifflichkeit "unmittelbar" bedeutet hierbei, dass eine bestimmte Menge des Rezirkulats der ersten Hydrolyseeinrichtung zum Beispiel direkt und damit unmittelbar zugeführt werden kann. Alternativ oder zusätzlich dazu kann aber auch vorgesehen sein, dass eine definierte Menge des Rezirkulats der ersten Hydrolyseeinrichtung "mittelbar" zugeführt wird, indem das Rezirkulat der aufgeschlossenen Biomasse stromauf der Hydrolyseeinrichtung zugeführt bzw. beigemischt wird. Bezüglich der zweiten Hydrolyseeinrichtung bedeutet dies, dass eine definierte Menge des Rezirkulats der zweiten Hydrolyseeinrichtung ebenfalls direkt und damit unmittelbar zugeführt werden kann. Alternativ oder zusätzlich dazu kann aber auch hier vorgesehen sein, dass eine definierte Menge des Rezirkulats der zweiten Hydrolyseeinrichtung mittelbar zugeführt wird, indem das Rezirkulat der von der ersten Separatoreinrichtung kommenden Feststoffphase stromauf der zweiten Hydrolyseeinrichtung beigemischt wird.

Das in der ersten Hydrolyseeinrichtung entstehende Hydrolysegas macht dabei nur einen geringen Anteil der gesamten Gasausbeute des erfindungsgemäßen Prozesses aus, in etwa 5%, um einen beispielhaften Wert zu nennen, wobei dieses Hydrolysegas im wesentlichen CO₂ sowie zu einem geringen Anteil H₂, CH₄ (das eigentliche "Biogas" bzw. Methan) und lediglich Spuren von H₂S aufweist. Der Methanschlupf in dieser ersten Hydrolysestufe ist somit vernachlässigbar gering. Das in der zweiten Hydrolyseeinrichtung entstehende Hydrolysegas macht ebenfalls wiederum nur einen geringen Anteil an der gesamten Gasausbeute des erfindungsgemäßen Prozesses aus, zum Beispiel in etwa 5% der Gesamtgasausbeute, um auch hier einen beispielhaften Wert zu nennen. Dieses Hydrolysegas weist zwar regelmäßig einen gegenüber dem Hydrolysegas der ersten Hydrolysestufe höheren Anteil an CH₄ auf, was aber immer noch vernachlässigbar gering ist. Der überwiegende Großteil des Gases, regelmäßig um die 90%, und damit der Großteil des Methans (CH₄) entsteht somit bei der erfindungsgemäßen Verfahrensführung erst im Bereich des mit den Flüssigphasen beschickten Endlagers bzw. Fermenters, so dass die eigentliche Biogasproduktion hier insoweit auf im Wesentlichen eine einzige Endlager- bzw. Fermenterstufe konzentriert werden kann. Zudem kann damit die Biogasproduktion mit der erfindungsgemäßen Lösung insgesamt sehr vorteilhaft anforderungs- bzw. lastabhängig gesteuert und/oder geregelt werden, was nachfolgend noch näher erläutert wird.

Mit einer derartigen Verfahrensführung wird weiter eine wesentliche höhere Effizienz bei der Umsetzung der Biomasse bzw. eine Steigerung der Biogasausbeute durch Erhöhung der Abbaugeschwindigkeit der Biomasse erzielt. So wird zum einen durch die der ersten Hydrolysestufe bzw. -einrichtung zugeführte, bereits aufgeschlossene bzw. wenigstens zum Teil aufgeschlossene Biomasse sichergestellt, dass die Biomasse unter optimalen Bedingungen hydrolysiert werden kann. Insbesondere kann hier die bereits aufgeschlossene Biomasse bei einem für die Hydrolysestufe besonders geeigneten pH-Wert hydrolysiert werden, was sich vorteilhaft auf den gesamten Prozess auswirkt. Durch die anschließende Phasentrennung in der Separatoreinrichtung wird weiter sichergestellt, dass der zweiten Hydrolyseeinrichtung nicht die gesamte hydrolysierte Biomasse (Feststoffphase und Flüssigphase) aus der ersten Hydrolysestufe zugeführt wird, sondern im Wesentlichen nur die in dieser Prozessstufe anfallende Feststoffphase, so dass die Hydrolyse in dieser zweiten Hydrolysestufe bzw. -einrichtung wesentlich schneller und gezielter ablaufen kann, wodurch die Verweilzeit der zu vergärenden Biomasse gegenüber herkömmlichen Blogasanlagen deutlich reduziert werden kann.

Die Flüssigphase aus der Phasentrennung mittels der ersten und der zweiten Separatoreinrichtung, die bevorzugt einen hohen Anteil an flüssigen Fettsäuren aufweist, wird dagegen wenigstens zum Teil in das Endlager bzw. den Fermenter eingebracht, wo diese Flüssigphase ebenfalls sehr schnell abgebaut werden kann. Dementsprechend muss dem Endlager bzw. Fermenter für den Vergärungsprozess wesentlich weniger Biomasse und vor allem wesentlich weniger Flüssigkeit, wie zum Beispiel Wasser, zugeführt werden, um eine - bezogen auf eine herkömmliche Biogasanlage mit mehreren Fermentern - gleiche Biogasausbeute zu erzielen. Dadurch kann das Endlager- bzw. Fermentervolumen insgesamt deutlich reduziert werden bzw. kann insgesamt Fermentervolumen eingespart werden, wodurch sich trotz hoher Biogasausbeute kleinvolumige bzw. kompakte Anlagen realisieren lassen. Die Hydrolysestufen selbst können aufgrund deren gezielter Auslegung und Abstimmung auf den Hydrolyseprozess ebenfalls wesentlich kleinvolumiger ausgelegt werden als Fermenterbehälter von eine vergleichbare Biogasausbeute erzielenden Biogasanlagen.

lnsbesondere durch die Rückführung eines aus dem Endlager bzw. dem Fermenter abgezogenen Rezirkulats lässt sich gemäß einer besonders bevorzugten Verfahrensführung eine Optimierung einer oder beider Hydrolysestufen, insbesondere im Hinblick auf den dort eingestellten pH-Wert erzielen. Denn mit dem rückgeführten Rezirkulat kann auf einfache Weise eine pH-Wertanhebung in der jeweiligen Hydrolysestufe bewirkt werden, während hingegen die Beschickung der ersten Hydrolysestufe mit der aufgeschlossenen Biomasse bzw. der zweiten Hydrolysestufe mit der separierten Feststoffphase jeweils eine pH-Wertminderung bewirkt. Dadurch lässt sich somit der pH-Wert einer oder beider Hydrolysestufe(n) auf einfache Weise stets in einem gewünschten pH-Wertbereich einstellen bzw. einregeln, wodurch eine optimale Hydrolyse der jeweils eingesetzten Biomasse in der jeweiligen Hydrolysestufe erfolgt. Dies trägt, wie bereits zuvor beschrieben, dazu bei, die Verweilzeiten sowohl in den Hydrolysestufen als auch in der Endlager- bzw. Fermenterstufe zu verringern, so dass sowohl die Hydrolyseeinrichtungen als auch die Endlager- bzw. Fermenterstufe entsprechend kleinvolumig und kompakt ausgelegt werden können. Es versteht sich von selbst, dass durch diese Reduzierung der Verweilzeiten auch eine Optimierung der Biogasausbeute insgesamt erzielt werden kann.

Die allgemeine Erfindungsidee wurde zuvor in Verbindung mit Begrifflichkeiten wie "Aufschlusseinrichtung", "Separatoreinrichtung", "Hydrotyseeinrichtung" und "Endlager" bzw. "Fermenter" beispielhaft näher erläutert. Es versteht sich von selbst, dass diese Anlagenbestandteile grundsätzlich ein- oder mehrstufig ausgebildet sein können, je nach Verfahrensführung und zu behandelnder Biomasse. Des Weiteren kann zum Beispiel die Aufschlusseinrichtung auf unterschiedliche Art und Weise gebildet sein, zum Beispiel durch einen Querstromzerspaner, einen Prallreaktor oder aber auch durch einen Schneckenextruder, um nur einige Beispiele zu nennen. Die Hydrolyseeinrichtung kann zum Beispiel durch jeden geeigneten Hydrolysator, zum Beispiel durch eine gasdichte Hydrolysegrube, gebildet sein. Als Separatoreinrichtung können beispielsweise Pressen, Zentrifugen oder Siebe eingesetzt werden, wobei selbstverständlich auch der Einsatz jeglicher anderer geeigneter Abscheider als Separatoreinrichtung möglich ist. Bei dem Endlager bzw. Fermenter kann es sich zum Beispiel um einen in üblicher Weise aufgebauten Reaktor handeln, der beispielsweise durch einen Rundbehälter mit Foliendach gebildet ist.

Wie bereits zuvor erwähnt, kann das zur ersten Hydrolysestufe rückgeführte Rezirkulat auf unterschiedliche Art und Weise in die erste Hydrolyseeinrichtung eingebracht werden. Besonders vorteilhaft ist hierbei eine Verfahrensführung, bei der zwischen der Aufschlusseinrichtung und der Hydrolyseeinrichtung eine Förder- und/oder Zudosiereinrichtung, insbesondere eine Pumpeinrichtung, zum Beispiel eine Schneckenpumpe, vorgesehen ist, mittels der die aufgeschlossene Biomasse, vorzugsweise unter Vermischung derselben mit dem ebenfalls der Förder- und/oder Zudosiereinrichtung oder stromauf der Förder- und/oder Zudosiereinrichtung zugeführten Rezirkulat zur ersten Hydrolyseeinrichtung gefördert wird. Mit einer derartigen Förder- und/oder Zudosiereinrichtung ist somit auf einfache Weise sichergestellt, dass sich das rückgeführte Rezirkulat sehr gut mit dem aufgeschlossenen Biomassematerial mischt. Anstelle der eben beispielhaft erwähnten Pumpeinrichtung könnte die Zuführung der aufgeschlossenen Biomasse zur Hydrolyseeinrichtung grundsätzlich aber auch mittels jeder anderen geeigneten Förder- und/oder Zudosiereinrichtung in analoger Weise erfolgen. Hierzu eignen sich zum Beispiel Förderbänder, Kettenförderer oder dergleichen Förder- und/oder Zudosiereinrichtungen, die dann die aufgeschlossene Biomasse zur Hydrolyseeinrichtung fördern, wo sie zum Beispiel mittels einer Einbringungsschnecke in die Hydrolyseeinrichtung eingebracht wird.

Wie ebenfalls bereits zuvor angedeutet, ist es für eine optimierte Verfahrensführung weiter besonders vorteilhaft, dass die zur ersten Hydrolyseeinrichtung rückgeführte Rezirkulatmenge und/oder die der ersten Hydrolyseeinrichtung zugeführte Menge an aufgeschlossener Biomasse in Abhängigkeit von einem vorgegebenen pH-Wert in der ersten Hydrolyseeinrichtung und/oder in Abhängigkeit von einem Füllstand der ersten Hydrolyseeinrichtung und/oder in Abhängigkeit von einer Verweilzeit (HRT = hydraulic retention time) des Hydrolysesubstrates in der ersten Hydrolyseeinrichtung vorgegeben wird. Die Vorgabe kann hier insbesondere durch entsprechende Steuerung bzw. Regelung der Stoffströme mittels einer Steuer- und/oder Regeleinrichtung der Biogasanlage erfolgen. Besonders bevorzugt ist hierbei eine Verfahrensführung, bei der der pH-Wert in der ersten Hydrolyseeinrichtung auf einen definierten pH-Wert, bevorzugt von 5 bis 7, höchst bevorzugt von 6,2 bis 6,7, eingeregelt wird. Denn dadurch lässt sich eine Verfahrensführung erzielen, bei der, bei einem einen vorgegebenen pH-Schwellwert unterschreitendem pH-Wert bzw. einer erfassten und/oder ermittelten Anhebnotwendigkeit des pH-Wertes, Rezirkulat der ersten Hydrolyseeinrichtung zudosiert oder die der ersten Hydrolyseeinrichtung zugeführte Rezirkulatmenge erhöht wird. Alternativ oder zusätzlich dazu kann auch die Menge an zugeführter aufgeschlossener Biomasse reduziert werden. Andererseits kann bei einem, einen vorgegebenen pH-Schwellwert überschreitenden pH-Wert bzw. einer erfassten und/oder ermittelten Absenknotwendigkeit des pH-Wertes, aufgeschlossene Biomasse zudosiert werden oder die Menge an zugeführter aufgeschlossener Biomasse erhöht werden. Alternativ oder zusätzlich kann auch vorgesehen werden, die Menge an rückgeführtem Rezirkulat zu reduzieren. Mit einer derartigen pHwertabhängigen und/oder füllstandsabhängigen und/oder verweilzeitabhängigen Steuerung bzw. Regelung der ersten Hydrolysestufe wird auf einfache Weise erreicht, dass eine optimale Hydrolyse des eingesetzten Biomassematerials mit erwünschter geringer Verweilzeit erfolgt. Die Verweilzeit wird dabei als "hydraulic retention time" (HRT = hydraulische Rückhaltezeit) gemessen bzw. angegeben. Dabei ist insbesondere eine konkrete Verfahrensführung vorteilhaft, bei der der Füllstand der ersten Hydrolyeeinrichtung durch Zudosierung von Rezirkulat und/oder aufgeschlossener Biomasse in die erste Hydrofyseeinrichtung so eingeregelt wird, dass das Hydrolysesubstrat in der ersten Hydrolyseeinrichtung bei einem definierten pH-Wert von 5 bis 7, vorzugsweise von 6,2 bis 6,7, eine Verweilzeit von lediglich 1 bis 8 Tagen, bevorzugt von lediglich 3 bis 5 Tagen, aufweist.

Besonders vorteilhaft ist eine erfindungsgemäße Verfahrensführung, bei der der Trockensubstanzanteil (TS-Anteil) der aufgeschlossenen Biomasse in der ersten Hydrolyseeinrichtung auf in etwa 7 bis 13%TS reduziert wird, vorzugsweise auf in etwa 10%TS reduziert wird. Hierdurch ergeben sich optimale Verhältnisse für die Phasentrennung in der nachgeschalteten Separatoreinrichtung im Hinblick auf die dieser wiederum nachfolgenden Hydrolyse- und Fermentationsstufen.

Besonders bevorzugt ist weiter eine Verfahrensführung, bei der der TS-Anteil der Feststoffphase nach Durchlaufen der der Hydrolyseeinrichtung nachgeschalteten ersten Separatoreinrichtung in etwa wenigstens 15%TS, bevorzugt in etwa zwischen 15 bis 30%TS und höchst bevorzugt in etwa 18 %TS beträgt. Bei derartigen TS-Anteilen der der ersten Hydrolyseeinrichtung zugeführten Biomasse lassen sich die Behälterinhalte am besten rühren.

Gemäß einer bevorzugten konkreten Verfahrensführung wird weiter (in einer analogen Weise zur zuvor beschriebenen Verfahrensführung der Rezirkulat-Rückführung zur ersten Hydrolysenstufe) vorgeschlagen, dass die zur zweiten Hydrolyseeinrichtung rückgeführte Rezirkulatmenge und/oder die der zweiten Hydrolyseeinrichtung zugeführte Menge an von der ersten Separatoreinrichtung kommender erster Feststoffphase in Abhängigkeit von einem vorgegebenen pH-Wert in der zweiten Hydrolyseeinrichtung und/oder in Abhängigkeit von einem Füllstand der zweiten Hydrolyseeinrichtung und/oder in Abhängigkeit von einer Verweilzeit (HRT = hydraulic retention time) des Hydrolysesubstrats in der zweiten Hydrolyseeinrichtung vorgegeben wird, insbesondere gesteuert und/oder geregelt mittels einer Steuer- und/oder Regeleinrichtung vorgegeben wird. Dadurch lässt sich auf einfache Weise eine optimale Einstellung der Hydrolysebedingungen für die in dieser Prozessphase regelmäßig noch in der Feststoffphase vorhandene und relativ schwer abbaubare Cellulose, Hemicellulose, Lignin oder dergleichen erreichen. Konkret kann hierzu gemäß einer besonders bevorzugten Ausgestaltung vorgesehen sein, dass der pH-Wert in der zweiten Hydrolyseeinrichtung auf einen definierten pH-Wert, bevorzugt von in etwa 6 bis 8,5, höchst bevorzugt von in etwa 7 bis 8, eingeregelt wird, insbesondere dergestalt, dass bei einem einen vorgegebenen pH-Schwellwert unterschreitendem pH-Wert bzw. einer erfassten und/oder ermittelten Anhebnotwendigkeit des pH-Wertes der zweiten Hydrolyseeinrichtung Rezirkulat zudosiert oder die der zweiten Hydrolyseeinrichtung zugeführte Rezirkulatmenge erhöht wird und/oder die Menge an zugeführter erster Feststoffphase reduziert wird. Alternativ dazu kann bei einem einen vorgegebenen pH-Schwellwert überschreitenden pH-Wert bzw. einer erfassten und/oder ermittelten Absenknotwendigkeit des pH-Wertes erste Feststoffphase zudosiert oder die Menge an zugeführter erster Feststoffphase erhöht werden und/oder die Menge an rückgeführtem Rezirkulat reduziert werden. Dabei ist insbesondere eine Verfahrensführung vorteilhaft, bei der der Füllstand der zweiten Hydrolyseeinrichtung durch Zudosierung von Rezirkulat und/oder erster Feststoffphase in die zweite Hydrolyseeinrichtung so eingeregelt wird, dass das Hydrolysesubstrat in der zweiten Hydrolyseeinrichtung bei einem definierten pH-Wert von in etwa 6 bis 8,5, vorzugsweise von in etwa 7 bis 8, eine Verweilzeit von lediglich noch 10 bis 50 Tagen, bevorzugt von lediglich noch 25 bis 35 Tagen, aufweist. Wie diese Werte zeigen lässt sich somit bei einer derartigen Verfahrensführung eine optimale Aufbereitung der Biomasse mit besonders geringen Verweilzeiten erzielen. Grundsätzlich wäre es zwar wünschenswert, wenn der pH-Wert der zweiten Hydrolysestufe in etwa gleich demjenigen der ersten Hydrolysestufe wäre, also zum Beispiel bei einem pH-Wert von ca. 6,2 bis 6,7 liegt, was jedoch biologisch oftmals nur schwer zu realisieren ist, da die Fermentation regelmäßig schon vor der Hydrolysierung der gesamten Biomasse einsetzt. Das heißt mit anderen Worten, dass der pH-Wert der zweiten Hydrolysestufe bevorzugt in etwa demjenigen der ersten Hydrolysestufe entsprechen sollte, jedoch aufgrund der schon einsetzenden Fermentation durch die lange Verweilzeit regelmäßig nicht auf diesem Niveau gehalten werden kann, ohne dass zu viel Verweilzeit (HRT = hydraulic retention time) eingebüßt wird, wenn zu viel rezirkuliert wird. Aus diesem Grund kann ein höherer pH-Wert in der zweiten Hydrolysestufe und damit schon eine gewisse Biogasproduktion akzeptiert werden, die sich jedoch nicht schädlich auswirkt. Konkret kann somit gemäß einer besonders bevorzugten konkreten Ausgestaltung vorgesehen sein, dass der pH-Wert in der zweiten Hydrolyseeinrichtung auf einen pH-Wert von in etwa 7 bis 8,5, höchst bevorzugt von in etwa 7 bis 8, eingeregelt wird.

Gegebenenfalls kann zwischen der ersten Separatoreinrichtung und der zweiten Hydrolyseeinrichtung eine weitere, dann zweite Aufschlusseinrichtung vorgesehen sein, mittels der die noch nicht aufgeschlossenen Feststoffe der von der ersten Separatoreinrichtung kommenden Feststoffphase wenigstens zum Teil aufgeschlossen werden. Es versteht sich von selbst, dass hierdurch der Wirkungsgrad der Hydrolyse in der zweiten Hydrolysestufe bzw. -einrichtung nochmals zusätzlich wesentlich erhöht werden kann.

Gemäß einer weiteren bevorzugten Ausgestaltung der erfindungsgemäßen Verfahrensführung wird zudem vorgeschlagen, dass die der zweiten Hydrolyseeinrichtung nachgeschaltete zweite Separatoreinrichtung so ausgelegt ist, dass der TS-Anteil der zweiten Flüssigphase in etwa 2 bis 6%TS, bevorzugt in etwa 3 bis 4%TS beträgt.

Gemäß einer weiteren besonders bevorzugten Verfahrensführung wird vorgeschlagen, dass die Biogasproduktion der Biogasanlage mittels der Beschickung des Endlagers bzw. Fermenters mit Flüssigphase gesteuert und/oder geregelt wird, insbesondere dergestalt, dass die Beschickung des Endlagers bzw. Fermenters mit Flüssigphase zurückgefahren wird, wenn weniger Biogas benötigt wird als aktuell erzeugt wird oder dass alternativ die Menge der dem Endlager bzw. dem Fermenter zugeführten Flüssigphase erhöht wird, wenn mehr Biogas benötigt wird als aktuell erzeugt wird. Mit einer derartigen Verfahrensführung kann auf den Einsatz von Biogas-Zwischenspeichern zum Beispiel ganz verzichtet werden oder können diese vom Volumen her deutlich reduziert werden, da in Abhängigkeit von zum Beispiel der Motorleistung und/oder einer Lastanforderung die Biogaszufuhr zum Motor geregelt wird. Beispielsweise kann in diesem Zusammenhang vorgesehen werden, dass bei einem aktuellen Beschickungsbedarf des Motors von 500m³ die Biogasproduktion durch entsprechende Steuerung der Flüssigphasenzudosierung zum Endlager bzw. Fermenter so geregelt wird, dass die genau benötigte Biogasmenge am Motor zur Verfügung steht.

Die vorstehende Aufgabe wird ferner durch eine Biogasanlage gemäß Anspruch 15 gelöst. Die sich hierdurch und mit den darauf rückbezogenen Unteransprüchen ergebenden Vorteile wurden bereits zuvor in Verbindung mit der ausführlichen Erläuterung der erfindungsgemäßen Verfahrensführung angegeben, so dass auf deren Wiederholung an dieser Stelle verzichtet wird und dementsprechend auf die zuvor gemachten Ausführungen verwiesen wird.

Lediglich ergänzend sei hier noch eine besonders bevorzugte Ausgestaltung erwähnt, gemäß der vorgeschlagen wird, dass die Hydrolyseeinrichtungen oder alternativ wenigstens eine der Hydrolyseeinrichtungen und der Fermenter bzw. das Endlager als Ring-in-Ring-Behälter ausgebildet sind, zum Beispiel dergestalt, dass ein erster Behälter im Inneren und bevorzugt beabstandet von einer Behälterwand eines zweiten Behälters angeordnet ist, oder ein erster Behälter im Inneren und bevorzugt beabstandet von einer Behälterwand eines zweiten Behälters angeordnet, der wiederum im Inneren und bevorzugt beabstandet von einer Behälterwand eines dritten Behälters angeordnet ist. Mit einem derartigen Aufbau wird ersichtlich eine besonders kompakte kleinbauende Anlage realisierbar.

Die Erfindung wird nachfolgend anhand einer Zeichnung beispielhaft näher erläutert.

Es zeigen:
- Fig. 1: eine Prinzipskizze einer beispielhaften erfindungsgemäßen Ausführungsform, und
- Fig. 2: schematisch eine alternative Ausgestaltung mit einer Ring-in-Ring-Konstruktion der Hydrolysestufen.

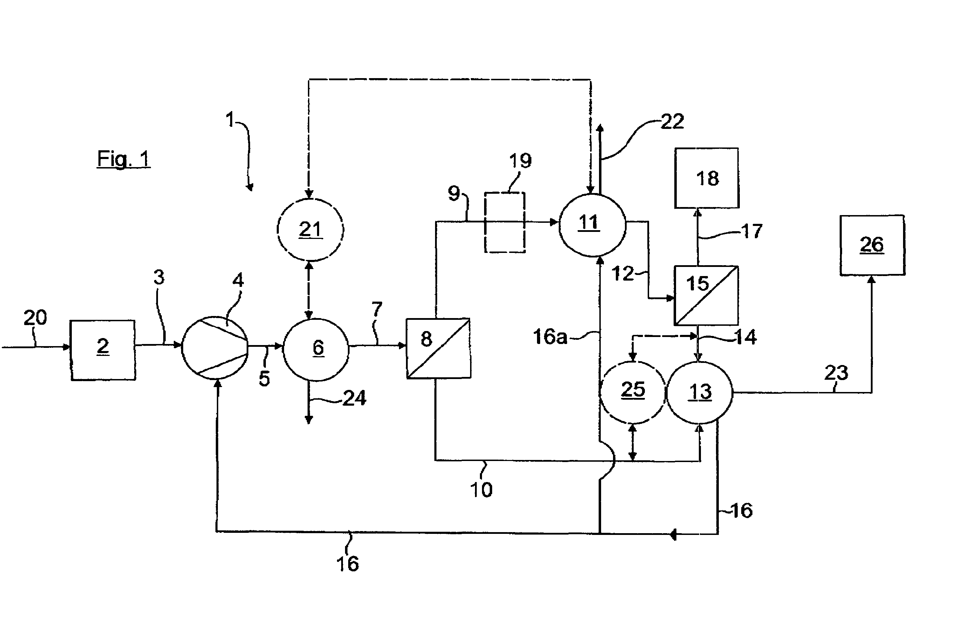

Fig. 1 zeigt beispielhaft eine mögliche konkrete Ausführungsform der erfindungsgemäßen Biogasanlage 1 mit einer ersten Aufschlusseinrichtung 2, die zum Beispiel durch einen Extruder, zum Beispiel einem Schneckenextruder oder dergleichen, gebildet ist und die mittels Biomasse 20, zum Beispiel mit nachwachsenden Rohstoffen (zum Beispiel Mais) sowie gegebenenfalls mit Gülle als Kosubstrat, beschickt wird. In der Aufschlusseinrichtung 2 findet ein wenigstens teilweiser Zellaufschluss der zugeführten Biomasse 20 statt. Die von der Aufschlusseinrichtung 2 abgezogene, aufgeschlossene Biomasse wird mittels einer Förder- und/oder Zudosiereinrichtung einer Hydrolyseeinrichtung 6 zugeführt. Die Zuführung erfolgt in der Fig. 1 lediglich beispielhaft über eine zum Beispiel durch eine Rohrleitung gebildete Pumpenleitung 3 einer zum Beispiel als Schneckenpumpe 4 ausgebildeten Pumpe 4. Dieser hier beispielhaft als Pumpe 4 ausgebildeten Förder- und/oder Zudosiereinrichtung wird ferner in nachfolgend noch näher beschriebener Weise mittels einer Rezirkulatleitung 16 ein Rezirkulat aus einem Endlager bzw. aus einem Fermenter 13 zugeführt, wobei die von der Aufschlusseinrichtung 2 kommende aufgeschlossene Biomasse und das Rezirkulat vor deren Zuführung zur Hydrolyseeinrichtung 6 bevorzugt gut miteinander vermischt werden, bevor das Rezirkulat und die aufgeschlossene Biomasse mittels einer Hydrolyseleitung 5 der zum Beispiel durch eine gasdichte Hydrolysegrube gebildeten ersten Hydrolyseeinrichtung 6 zugeführt wird. In der Hydrolyseeinrichtung 6 wird dann das Gemisch aus aufgeschlossener Biomasse und rückgeführtem Rezirkulat, vorzugsweise bei einem pH-Wert zwischen 6,2 und 6,7, für eine maximale Verweilzeit zwischen zum Beispiel 3 bis 5 Tagen hydrolysiert wird.

Die Hydrolyse in der ersten Hydrolyseeinrichtung 6 wird dabei von einer Steuer- und/oder Regeleinrichtung 21 überwacht, mittels der sowohl die Menge der der ersten Hydrolyseeinrichtung 6 zugeführten bzw. zuführbaren aufgeschlossenen Biomasse als auch die Menge des rückgeführten bzw. rückführbaren Rezirkulats so gesteuert bzw. geregelt wird, dass sich in dieser ersten Hydrolysestufe stets der gewünschte pH-Wert, insbesondere ein pH-Wert zwischen 6,2 und 6,7, einregelt, und zwar vorzugsweise unter gleichzeitiger Kontrolle des Füllstandsniveaus in der ersten Hydrolyseeinrichtung 6, um die gewünschte Verweilzeit von zum Beispiel maximal 5 Tagen zu erzielen.

Anstelle der Pumpe 4 könnte die Zuführung der aufgeschlossenen Biomasse zur Hydrolyseeinrichtung 6 grundsätzlich aber auch mittels jeder anderen geeigneten Förder- und/oder Zudosiereinrichtung in analoger Weise erfolgen. Hierzu eignen sich zum Beispiel Förderbänder, Kettenförderer oder dergleichen Förder- und/oder Zudosiereinrichtungen, die dann die aufgeschlossene Biomasse zur Hydrolyseeinrichtung 6 fördern, wo sie zum Beispiel mittels einer Einbringungsschnecke in die Hydrolyseeinrichtung 6 eingebracht wird.

Ferner kann von der ersten Hydrolyseeinrichtung 6 ein Hydrolysegas über eine Gasleitung 24 abgezogen werden.

Am Ende der ersten Hydrolysestufe wird mittels einer Separatorleitung 7 ein hydrolysierter Biomasse-Stoffstrom von bevorzugt in etwa 10% TS abgezogen und einem ersten Separator 8 zugeführt, in dem eine Phasentrennung in eine feststoffhaltige Feststoffphase mit bevorzugt 18% TS und in eine im Wesentlichen aus flüssigen Fettsäuren bestehende Flüssigphase erfolgt.

Die im ersten Separator 8 erhaltene Feststoffphase (Cellulose, Hemicellulose, Lignin etc.) wird über eine Feststoffphasenleitung 9 einer zweiten Hydrolyseeinrichtung 11 zugeführt, während die flüssigen Fettsäuren der Flüssigphase über eine Flüssigphasenleitung 10 dem bereits zuvor erwähnten Fermenter 13 zugeführt werden, der der zweiten Hydralyseeinrichtung 11 nachgeschaltet ist. In der zweiten Hydrolyseeinrichtung 11, die zum Beispiel analog zur ersten Hydrolyseeinrichtung durch eine Hydrolysegrube oder dergleichen ausgebildet sein kann, wird die Feststoffphase vorzugsweise bei einem pH-Wert von in etwa 6 bis 8,5, vorzugsweise von in etwa 7 bis 8, für eine Verweilzeit von 10 bis 50 Tagen, bevorzugt von 25 bis 35 Tagen, weiter hydrolysiert. Die Hydrolyse in der zweiten Hydrolyseeinrichtung 11 wird dabei ebenfalls von der Steuer- und/oder Regeleinrichtung 21 überwacht, mittels der sowohl die Menge der der zweiten Hydrolyseeinrichtung 11 zugeführten bzw. zuführbaren Feststoffphase als auch die Menge des zur zweiten Hydrolyseeinrichtung 11 rückgeführten bzw. rückführbaren Rezirkulats, das mittels einer von der Rezirkulatleitung 16 abzweigenden und Bestandteil derselben bildenden Zweigleitung 16a zuführbar ist, so gesteuert bzw. geregelt wird, dass sich in dieser zweiten Hydrolysestufe stets der gewünschte pH-Wert, insbesondere ein pH-Wert zwischen 7,5 und 8,5, einregelt, und zwar vorzugsweise unter gleichzeitiger Kontrolle des Füllstandsniveaus in der zweiten Hydrolyseeinrichtung 11, um die gewünschte Verweilzeit von zum Beispiel maximal 35 Tagen zu erzielen.

Das in der zweiten Hydrolyseeinrichtung 11 erzeugte Hydrolysegas wird hier beispielhaft über eine Gasleitung 22 abgezogen.

Die hydrolysierte Biomasse aus der zweiten Hydrolyseeinrichtung 11 wird wenigstens zum Teil über eine zweite Separatorleitung 12 einem zweiten Separator 15 zugeführt, in dem die hydrolysierte Biomasse einer Phasentrennung unterzogen wird, wobei die so erhaltene und im Wesentlichen nicht mehr abbaubare Stoffe (Lignin, Lignocellulose oder dergleichen) enthaltene Feststoffphase einem Stoffrestelager 18 zugeführt wird, und zwar mittels der in der Fig. 1 gezeigten Leitung 17. Die Flüssigphase wird dagegen über eine Flüssigphasenleitung 14 ebenfalls dem Fermenter 13 zugeführt, in dem nun die schnell abbaubaren Flüssigphasen der beiden Separatoren 8 und 15 unter Biogaserzeugung schnell fermentiert werden.

Der zweite Separator 15 ist bevorzugt so ausgelegt, dass das Rezirkulat einen TS-Anteil von 3 bis 4% TS aufweist.

Von dem Fermenter 13 zweigt nunmehr die bereits zuvor beschriebene Rezirkulatleitung 16 ab, mittels der Rezirkulat aus dem Fermenter 13, das regelmäßig einen pH-Wert von größer 7, insbesondere von 8 bis 8,5, aufweist, in der zuvor beschriebenen Weise zur pH-Wertregulierung der ersten und/oder zweiten Hydrolyseeinrichtung 6, 11 rückgeführt wird.

Das im Fermenter 13 erzeugte, äußerst methanreiche Biogas wird hier über eine Gasleitung 23 abgezogen und beispielsweise dem hier lediglich schematisch dargestellten Gasmotor 26 zugeführt.

Besonders bevorzugt findet die Biogasproduktion in der Biogasanlage 1 hier mittels der Beschickung des Fermenters 13 mit Flüssigphase gesteuert und/oder geregelt mittels einer hier lediglich schematisch und strichliert dargestellten Steuer- und/oder Regeleinrichtung 25 (die genauso gut aber auch Bestandteil der Steuer- und/oder Regeleinrichtung 21 sein kann) statt, und zwar dergestalt, dass die Beschickung des Fermenters 13 mit Flüssigphase von dem ersten Separator 8 und/oder von dem zweiten Separator 15 gedrosselt bzw. zurückgehalten wird, wenn weniger Biogas benötigt wird als aktuell erzeugt wird. Andererseits kann mittels der Steuer- und/oder Regeleinrichtung 25 die Menge der dem Fermenter 13 zugeführten Flüssigphase von dem ersten Separator 8 und/oder von dem zweiten Separator 15 erhöht werden, wenn mehr Biogas benötigt wird als aktuell erzeugt wird.

Auch wenn hier vorstehend vom Fermenter 13 gesprochen wird, muss es sich hierbei, wie bereits zuvor erwähnt, nicht zwangsläufig um einen Fermenter handeln, sondern kann alternativ auch ein vorzugsweise beheiztes Endlager anstelle des Fermenters verwendet werden, da die vor allem Fettsäuren als flüssige Phase der Separation sehr schnell vergären können und somit der Einsatz eines Fermenters unter Umständen gar nicht mehr notwendig ist.

An dieser Stelle sei erwähnt, dass die Begrifflichkeit "Leitung" im vorliegenden Fall ausdrücklich in einem weiten Sinne zu verstehen ist und nicht auf Rohrleitungen als solche beschränkt ist. Das heißt, dass selbstverständlich sämtliche geeigneten Fördereinrichtungen bzw. Transporteinrichtungen für den Transport bzw. die Förderung der Stoffströme von der einen Baueinheit zu der anderen Baueinheit eingesetzt werden können. Die Begrifflichkeit "Leitung" wurde hier ausdrücklich zur prinzipiellen Beschreibung der Stoffströme und aus Gründen der besseren Übersichtlichkeit verwendet. Ferner können, insbesondere zur Steuerung bzw. Regelung der Stoffströme bzw. für die Zufuhr und Absperrung von Stoffströmen, Schieber, Klappen, Ventile, Pumpen oder dergleichen vorgesehen sein.

Des Weiteren kann, wie dies in der Fig. 1 lediglich schematisch und strichliert eingezeichnet ist, zwischen dem ersten Separator 8 und der zweiten Hydrolyseeinrichtung 11 eine zweite Aufschlusseinrichtung 19 vorgesehen sein, mittels der zum Beispiel bei Bedarf und optional die der zweiten Hydrolyseeinrichtung 11 zugeführte Feststoffphasencharge weiter aufgeschlossen werden kann, sofern dies notwendig sein sollte.

Mit einer derartigen erfindungsgemäßen pH-wertgesteuerten bzw. -geregelten Hydrolysestufe wird in Verbindung mit einer Separierung der hydrolysierten Biomasse vor dem ersten Fermenter und einer weiteren Separierung und Rückführung eines Rezirkulats eine Verfahrensführung und eine Biogasanlage 1 zur Verfügung gestellt, mittels der sowohl die Biogasausbeute als auch die Abbaugeschwindigkeit wesentlich erhöht werden kann.

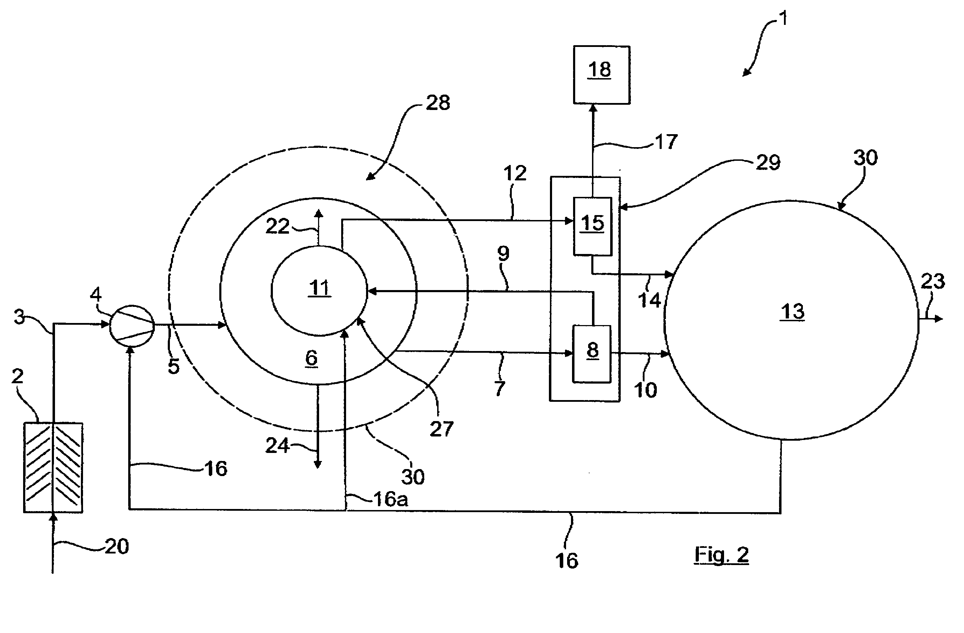

Gegenüber herkömmlichen Biogasanlagen lassen sich daher wesentlich kompaktere und kleinere Volumina der Fermenterbehälter erzielen, was insbesondere auch aus der Fig. 2 hervorgeht, in der die erste und zweite Hydrolyseeinrichtung 6, 11 als Ring-in-Ring-Behälter ausgebildet sind. Konkret ist hier ein Bestandteil der zweiten Hydrolyseeinrichtung 11 ausbildender zweiter Hydrolysebehälter 27 im Inneren und hier konzentrisch sowie beabstandet von einer Behälterwand eines Bestandteil der ersten Hydrolyseeinrichtung 6 bildenden ersten Hydrolysebehälters 28 angeordnet. Auch wenn die kreisringförmige Geometrie der beiden Hydrolysebehälter 27, 28 die bevorzugte Geometrie darstellt, versteht es sich, dass diese auch andere Geometrien, zum Beispiel rechteckförmige Geometrien aufweisen könnten.

Weiter können auch die beiden Separatoren zum Beispiel in einem gemeinsamen Container 29 angeordnet sein, wodurch ebenfalls eine kompakte Anordnung erzielt wird. Der Fermenter 13 ist hier zwar mit einem separaten Fermenterbehälter 30 dargestellt, könnte aber gemäß einer in der Fig. 2 lediglich strichliert dargestellten Variante ebenfalls als weiterer Ringbehälter um die beiden anderen Ringbehälter 27, 28 angeordnet sein, was eine noch kompaktere Anordnung und damit eine insgesamt kleinbauende Biogasanlage ergibt.

## Patentansprüche

1. Verfahren zur Erzeugung von Biogas aus Biomasse, bei dem die Biomasse (20) in einem ersten Schritt einer Aufschlusseinrichtung (2) zugeführt wird, in der ein Zellaufschluss erfolgt und die zugeführte Biomasse wenigstens teilweise aufgeschlossen wird,
wobei anschließend die aufgeschlossene Biomasse wenigstens zum Teil einer ersten Hydrolyseeinrichtung (6) zugeführt wird, in der die aufgeschlossene Biomasse wenigstens teilweise hydrolysiert wird,
wobei anschließend die in der ersten Hydrolyseeinrichtung (6) wenigstens teilweise hydrolysierte Biomasse wenigstens zum Teil einer ersten Separatoreinrichtung (8) zugeführt wird, in der eine Auftrennung in eine feststoffhaltige Feststoffphase und in eine Flüssigphase durchgeführt wird,
wobei die Feststoffphase wenigstens zum Teil einer zweiten Hydrolyseeinrichtung (11) zugeführt wird, in der die Feststoffphase wenigstens teilweise hydrolysiert wird,
wobei anschließend die in der zweiten Hydrolyseeinrichtung (6) wenigstens teilweise hydrolysierte Biomasse wenigstens zum Teil einer zweiten Separatoreinrichtung (15) zugeführt wird, in der eine zweite Feststoffphase und eine zweite Flüssigphase abgetrennt wird, und
wobei sowohl die mittels der ersten Separatoreinrichtung (8) abgetrennte erste Flüssigphase als auch die mittels der zweiten Separatoreinrichtung (15) abgetrennte zweite Flüssigphase wenigstens zum Teil einem Endlager und/oder Fermenter (13) zugeführt wird

2. Verfahren nach Anspruch 1, **dadurch gekennzeichnet, dass** aus dem Endlager und/oder Fermenter (13) zu definierten Zeiten und/oder in definierter Menge ein Rezirkulat (16) abgezogen und der ersten Hydrolyseeinrichtung (6) und/oder der zweiten Hydrolyseeinrichtung (11) mittelbar und/oder unmittelbar zugeführt wird.

3. Verfahren nach Anspruch 2, **dadurch gekennzeichnet, dass** eine definierte Menge des Rezirkulats der ersten Hydrolyseeinrichtung (6) unmittelbar zugeführt wird und/oder dass eine definierte Menge des Rezirkulats der ersten Hydrolyseeinrichtung (6) mittelbar zugeführt wird, indem das Rezirkulat der aufgeschlossenen Biomasse stromauf der ersten Hydrolyseeinrichtung (6) zugeführt wird.

4. Verfahren nach einem der vorhergehenden Ansprüche, **dadurch gekennzeichnet, dass** zwischen der Aufschlusseinrichtung (2) und der ersten Hydrolyseeinrichtung (6) eine Förder- und/oder Zudosiereinrichtung (4), insbesondere eine Pumpeinrichtung, vorgesehen ist, mittels der die aufgeschlossene Biomasse, vorzugsweise unter Vermischung derselben mit dem ebenfalls der Förder- und/oder Zudosiereinrichtung (4) oder stromauf der Förder- und/oder Zudosiereinrichtung (4) zugeführten Rezirkulat, zur ersten Hydrolyseeinrichtung (6) gefördert wird.

5. Verfahren nach einem der Ansprüche 2 bis 5, **dadurch gekennzeichnet, dass** eine definierte Menge des Rezirkulats der zweiten Hydrolyseeinrichtung (11) unmittelbar zugeführt wird und/oder dass eine definierte Menge des Rezirkulats der zweiten Hydrolyseeinrichtung (11) mittelbar zugeführt wird, indem das Rezirkulat der von der ersten Separatoreinrichtung (8) kommenden Feststoffphase beigemischt wird.

6. Verfahren nach einem der Ansprüche 2 bis 5, **dadurch gekennzeichnet, dass** die zur ersten Hydrolyseeinrichtung (6) rückgeführte Rezirkulatmenge und/oder die der ersten Hydrolyseeinrichtung (6) zugeführte Menge an aufgeschlossener Biomasse in Abhängigkeit von einem vorgegebenen pH-Wert in der ersten Hydrolyseeinrichtung (6) und/oder in Abhängigkeit von einem Füllstand der ersten Hydrolyseeinrichtung (6) und/oder in Abhängigkeit von einer Verweilzeit des Hydrolysesubstrats in der ersten Hydrolyseeinrichtung (6) vorgegeben wird, insbesondere gesteuert und/oder geregelt mittels einer Steuer- und/oder Regeleinrichtung (21) vorgegeben wird.

7. Verfahren nach Anspruch 6, **dadurch gekennzeichnet, dass** der pH-Wert in der ersten Hydrolyseeinrichtung (6) auf einen definierten pH-Wert, bevorzugt von 5,0 bis 7,0, höchst bevorzugt von 6,2 bis 6,7, eingeregelt wird, insbesondere dergestalt, dass
a) bei einem einen vorgegebenen pH-Schwellwert unterschreitendem pH-Wert bzw. einer erfassten und/oder ermittelten Anhebnotwendigkeit des pH-Wertes Rezirkulat der ersten Hydrolyseeinrichtung (6) zudosiert oder die der ersten Hydrolyseeinrichtung (6) zugeführte Rezirkulatmenge erhöht wird und/oder die Menge an zugeführter aufgeschlossener Biomasse reduziert wird,
oder
b) bei einem einen vorgegebenen pH-Schwellwert überschreitenden pH-Wert bzw. einer erfassten und/oder ermittelten Absenknotwendigkeit des pH-Wertes aufgeschlossene Biomasse zudosiert oder die Menge an zugeführter aufgeschlossener Biomasse erhöht wird und/oder die Menge an rückgeführtem Rezirkulat reduziert wird.

8. Verfahren nach Anspruch 6 oder 7, **dadurch gekennzeichnet, dass** der Füllstand der ersten Hydrolyseeinrichtung (6) durch Zudosierung von Rezirkulat und/oder aufgeschlossener Biomasse in die erste Hydrolyseeinrichtung (6) so eingeregelt wird, dass das Hydrolysesubstrat in der ersten Hydrolyseeinrichtung (6) bei einem definierten pH-Wert von 5,0 bis 7,0, vorzugsweise von 6,2 bis 6,7, eine Verweilzeit von 1 bis 8 Tagen, bevorzugt von 3 bis 5 Tagen, aufweist.

9. Verfahren nach einem der vorhergehenden Ansprüche, **dadurch gekennzeichnet, dass** der Trockensubstanz (TS)-Anteil der aufgeschlossenen Biomasse in der ersten Hydrolyseeinrichtung (6) auf in etwa 7 bis 13% TS reduziert wird, vorzugsweise auf in etwa 10% TS reduziert wird und/oder dass der TS-Anteil der Feststoffphase nach Durchlaufen der der ersten Hydrolyseeinrichtung (6) nachgeschalteten ersten Separatoreinrichtung (8) in etwa wenigstens 15%TS, bevorzugt in etwa zwischen 15 bis 30%TS, höchst bevorzugt in etwa 18 %TS beträgt.

10. Verfahren nach einem der Ansprüche 2 bis 9, **dadurch gekennzeichnet, dass** die zur zweiten Hydrolyseeinrichtung (11) rückgeführte Rezirkulatmenge und/oder die der zweiten Hydrolyseeinrichtung (11) zugeführte Menge an von der ersten Separatoreinrichtung (8) kommender erster Feststoffphase in Abhängigkeit von einem vorgegebenen pH-Wert in der zweiten Hydrolyseeinrichtung (11) und/oder in Abhängigkeit von einem Füllstand der zweiten Hydrolyseeinrichtung (6) und/oder in Abhängigkeit von einer Verweilzeit (HRT = hydraulic retention time) des Hydrolysesubstrats in der zweiten Hydrolyseeinrichtung (11) vorgegeben wird, insbesondere gesteuert und/oder geregelt mittels einer Steuer- und/oder Regeleinrichtung (21) vorgegeben wird.

11. Verfahren nach Anspruch 10, **dadurch gekennzeichnet, dass** der pH-Wert in der zweiten Hydrolyseeinrichtung (11) auf einen definierten pH-Wert, bevorzugt von in etwa 6 bis 8,5, bevorzugt von in etwa 7 bis 8,5, höchst bevorzugt von in etwa 7 bis 8, eingeregelt wird, insbesondere dergestalt, dass
a) bei einem einen vorgegebenen pH-Schwellwert unterschreitendem pH-Wert bzw. einer erfassten und/oder ermittelten Anhebnotwendigkeit des pH-Wertes Rezirkulat der zweiten Hydrolyseeinrichtung (11) zudosiert oder die der zweiten Hydrolyseeinrichtung (11) zugeführte Rezirkulatmenge erhöht wird und/oder die Menge an zugeführter erster Feststoffphase reduziert wird,
oder
b) bei einem einen vorgegebenen pH-Schwellwert überschreitenden pH-Wert bzw. einer erfassten und/oder ermittelten Absenknotwendigkeit des pH-Wertes erste Feststoffphase zudosiert oder die Menge an zugeführter erster Feststoffphase erhöht wird und/oder die Menge an rückgeführtem Rezirkulat reduziert wird.

12. Verfahren nach Anspruch 10 oder 11, **dadurch gekennzeichnet, dass** der Füllstand der zweiten Hydrolyseeinrichtung (11) durch Zudosierung von Rezirkulat und/oder erster Feststoffphase in die zweite Hydrolyseeinrichtung (11) so eingeregelt wird, dass das Hydrolysesubstrat in der zweiten Hydrolyseeinrichtung (11) bei einem definierten pH-Wert von in etwa 7 bis 8,5, vorzugsweise von in etwa 7 bis 8, eine Verweilzeit von 10 bis 50 Tagen, bevorzugt von 25 bis 35 Tagen, aufweist.

13. Verfahren nach einem der vorhergehenden Ansprüche, **dadurch gekennzeichnet, dass** zwischen der ersten Separatoreinrichtung (8) und der zweiten Hydrolyseeinrichtung (11) eine weitere zweite Aufschlusseinrichtung (19) vorgesehen ist, mittels der die noch nicht aufgeschlossenen Feststoffe der von der ersten Separatoreinrichtung (8) kommenden Feststoffphase wenigstens zum Teil aufgeschlossen werden und/oder dass die zweite Separatoreinrichtung (15) so ausgelegt ist, dass der Trockensubstanz(TS)-Anteil der zweiten Flüssigphase in etwa 2 bis 6% TS, bevorzugt in etwa 3 bis 4%TS beträgt.

14. Verfahren nach einem der vorhergehenden Ansprüche, **dadurch gekennzeichnet, dass** die Biogasproduktion der Biogasanlage (1) mittels der Beschickung des Endlagers und/oder Fermenters (13) mit Flüssigphase gesteuert und/oder geregelt wird, insbesondere dergestalt, dass die Beschickung des Endlagers und/oder Fermenters (13) mit Flüssigphase zurückgefahren wird, wenn weniger Biogas benötigt wird als aktuell erzeugt wird oder dass die Menge der dem Endlager und/oder Fermenter (13) zugeführten Flüssigphase erhöht wird, wenn mehr Biogas benötigt wird als aktuell erzeugt wird.

15. Biogasanlage zur Erzeugung von Biogas, insbesondere zur Durchführung eines Verfahrens nach einem der vorhergehenden Verfahrensansprüche,
mit einer Aufschlusseinrichtung (2), der eine Förder- und/oder Zudosiereinrichtung (4) nachgeschaltet ist,
mit einer der Förder- und/oder Zudosiereinrichtung (4) und der Aufschlusseinrichtung (2) nachgeschalteten ersten Hydrolyseeinrichtung (6), der wiederum eine erste Separatoreinrichtung (8) nachgeschaltet ist,
wobei von der ersten Separatoreinrichtung (8) eine Feststoffphasenleitung (9), die zu einer zweiten Hydrolyseeinrichtung (11) geführt ist, und weiter eine Flüssigphasenleitung (10) abzweigt, die zu wenigstens einem Endlager und/oder Fermenter (13) geführt ist,
mit einer der zweiten Hydrolyseeinrichtung (11) nachgeschalteten zweiten Separatoreinrichtung (15), von der eine weitere Flüssigphasenleitung (14) abzweigt, die ebenfalls zu dem wenigstens einen Endlager und/oder Fermenter (13) geführt ist.

16. Biogasanlage nach Anspruch 15, **dadurch gekennzeichnet, dass** wenigstens eine von dem Endlager und/oder Fermenter (13) abzweigenden Rezirkulatleitung (16) vorgesehen ist, die mit der ersten und/oder zweiten Hydrolyseeinrichtung (11) mittelbar oder unmittelbar strömungsverbunden ist, und/oder dass eine Steuer- und/oder Regeleinrichtung (21) vorgesehen ist, mittels der die Stoffströme von und zu den einzelnen Einrichtungen der Biogasanlage (1) in Abhängigkeit von einem pH-Wert der ersten und/oder zweiten Hydrolyseeinrichtung (6, 11) und/oder in Abhängigkeit von einem Füllstand der ersten und/oder zweiten Hydrolyseeinrichtung (6, 11) und/oder in Abhängigkeit von einer Verweilzeit des Hydrolysesubstrats in der ersten und/oder zweiten Hydrolyseeinrichtung (6, 11) steuer- und/oder regelbar ist.

17. Biogasanlage nach Anspruch 15 oder 16, **dadurch gekennzeichnet, dass** die beiden Hydrolyseeinrichtungen (6, 11) oder wenigstens eine der beiden Hydrolyseeinrichtungen (6, 11) und das Endlager bzw. der Fermenter (13) als Ring-in-Ring-Behäiter ausgebildet sind.
